# EUROPEAN PATENT SPECIFICATION

(11) **EP 0 707 864 B1**
(45) Date of publication and mention of the grant of the patent: **28.12.2005**
(21) Application number: 95202755.5
(22) Date of filing: 12.10.1995
(51) Int. Cl.: A61M 25/10, A61F 2/06

(54) **Balloon catheter with several balloons**
Ballon-Katheter mit mehreren Ballonen
Cathéter à ballon ayant plusieurs ballons

(30) Priority: 21.10.1994 NL 9401759
(43) Date of publication of application: 24.04.1996
(73) Proprietor: CORDIS EUROPA N.V., NL-9301 LJ Roden (NL); Sheiban, Imad, 37126 Verona (IT)
(72) Inventor: Sheiban, Imad, I-37126 Verona (IT); Nap, Cornelis Philipus, NL-9345 AG Zevenhuizen (NL); Van Werven-Franssen, Gerda Hendrika Maria, NL-9302 EK Roden (NL)
(74) Representative: 't Jong, Bastiaan Jacobus

(56) References cited:
- EP-A- 0 260 107
- EP-A- 0 345 051
- DE-A- 2 659 238
- DE-A- 3 833 359
- US-A- 5 226 889
- US-A- 5 358 487

## Description

The invention relates to a catheter as defined in the introductory part of claim 1.

Such a catheter is known from the American patent specification 5 226 889. The relatively distal balloon is in this case a dilatation balloon, whereas the relatively proximal balloon is used for expanding a stent. This stent is arranged in compressed state around the relatively proximal balloon and thus introduced into the body of the patient. By expanding the balloon, the stent, which will subsequently remain behind inside the body of the patient, will also expand.

The European patent specifications 0345051 and 0260107 also describe catheters having several balloons. In EP 0345051 the distal balloon is note pliable than the proximal balloon.

Other types of catheters with several balloons comprise for instance an occlusion balloon as relatively distal balloon and a dilatation balloon as relatively proximal balloon.

The object of the invention is to provide an improved catheter of the type as described in the preamble.

This object is achieved with the features of the characterizing portion of claim 1.

A suitable embodiment is characterised in claim 2. The material of which the different balloon members have been made can be the same, whereby the required variation in pliability is achieved by employing different degrees of wall thickness.

As a result it will for instance be possible to make a dilatation balloon of a catheter more pliable than a balloon used for the purpose of fitting a stent. The greater pliability of the dilatation balloon allows for the application of a more uniform load on the wall of the vessel to be dilated, whereas for expanding a stent on the other hand a uniform expansion of the stent is more desirable than the application of a uniform load. This is achieved by making the balloon concerned less pliable.

Another suitable possibility is characterised in claim 3.

The invention is particularly suited for application with a catheter of the type which is to be used for the implantation of a stent, as has been mentioned above.

A further development is characterised in claim 5. A balloon member with a greater pliability will in general be used at lower pressures than a less pliable balloon member. Consequently great operational safety of the catheter according to the invention is achieved by employing the measure as set out in claim 5.

In the example of the catheter used for stent implantation, the relatively distal balloon is used, as has been mentioned before, for dilatation and requires less operating pressure than the relatively proximal balloon. The operating pressure of the dilatation balloon lies between 8-10 bar. The operating pressure of the balloon used for expanding the stent lies for instance between 14-16 bar.

In addition to the methods for realising the different degrees of pliability of the different balloon members mentioned already, the desired degrees of pliability in the different balloon members can also be achieved by employing suitable manufacturing processes.

The invention will be explained in greater detail in the following description with reference to the attached drawings.
- Figure 1: shows a partly broken away perspective view of a catheter according to the invention.
- Figure 2: shows a partly cut away view of the distal end of the catheter of fig. 1.
- Figure 3: shows a view corresponding to figure 2 during the second step of the treatment.

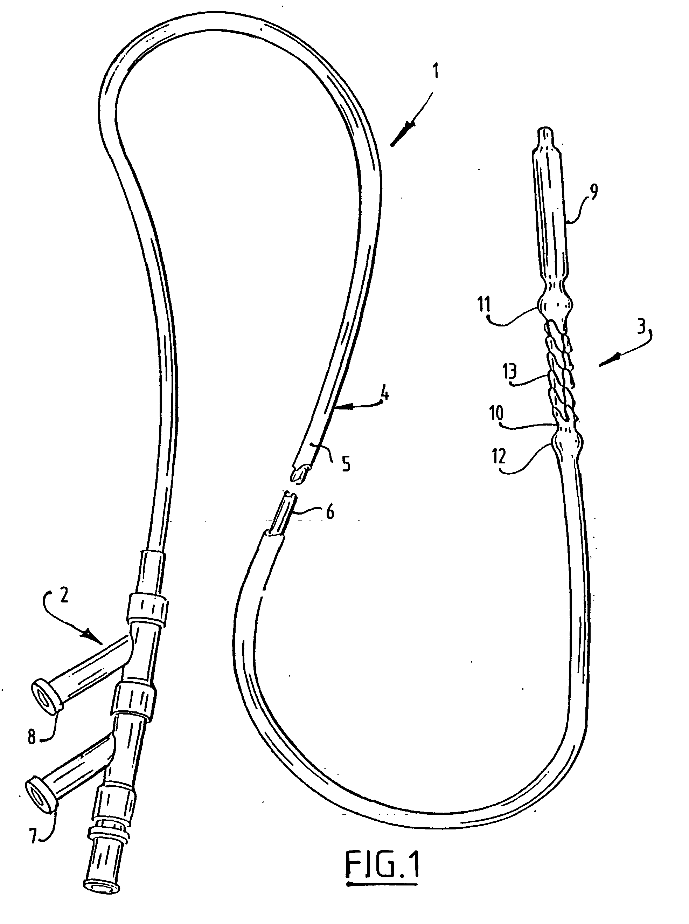

The catheter 1 shown in figure 1 comprises a tube-like basic body 4, which in this case has been made up of an outer tube-like element 5 and an inner tube-like element 6 received in a lumen thereof. The inner tube-like element 6 has two lumens 16, 17. With the embodiment shown, two balloon members 9, 10 have been arranged at the distal end 3 of the catheter 1. At the proximal end 2, two connecting members 7, 8 have been arranged which are connected to the balloon member 9 and the balloon member 10 respectively. By supplying medium under pressure via the connectors 7 or 8, the balloon members 9 or 10 can be expanded. The connection between the connecting member 7 and the balloon member 9 runs via the lumen 17 of the inner tube-like element 6 and the connection between the connecting member 8 and the balloon member 10 runs via the interspace 20 between the inner tube-like element 6 and the outer tube-like element 5. The lumen 16 is for receiving a guide wire.

A compressed stent 13 has been arranged around the balloon 10. The stent 13 is enclosed in axial direction on the catheter 1 by bulges 11 and 12, so that it cannot slide off the balloon 10.

As will be explained in greater detail below, the balloon member 9 is used for dilating a narrowed blood vessel and the balloon 10 for expanding the stent 13. According to the invention the balloons 9 and 10 possess different degrees of pliability. More in particular, the dilatation balloon 9 is more pliable than the balloon 10 used for expanding the stent 13.

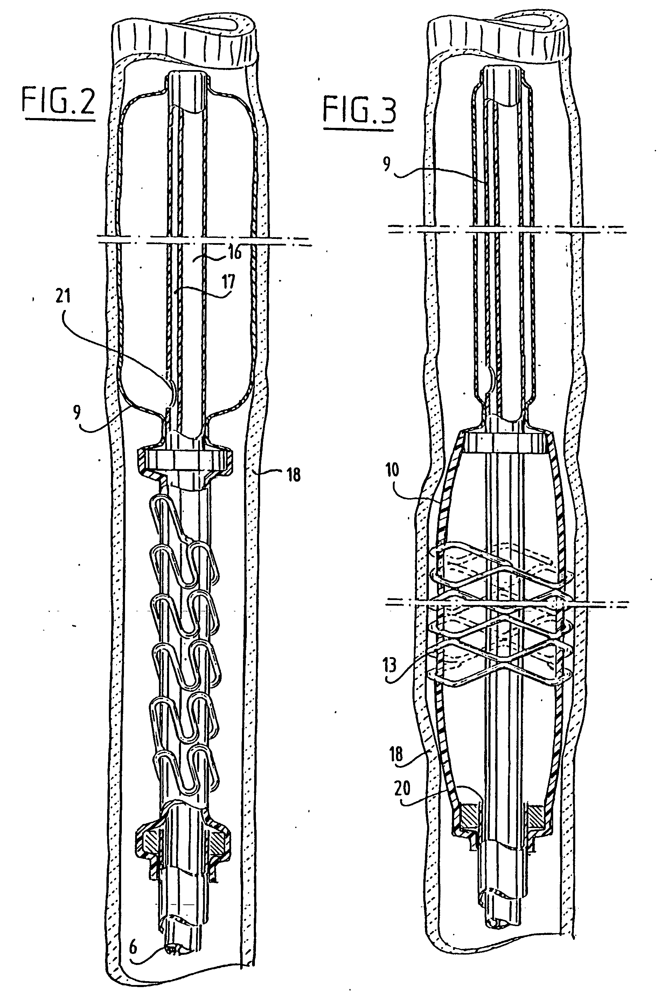

During treatment, the catheter 1 is first advanced into the blood vessel 18 that far, that the first balloon 9 is situated at the narrowed section of the vessel to be dilated. Next a medium under pressure is supplied via the connecting member 7 and through the lumen 17 of the inner tube-like element 6. This medium under pressure flows via the opening 21 in the wall of the inner tube-like member into the balloon 9, which will expand as a result. Consequently the blood vessel 18 will be dilated (fig. 2).

Because of the relatively great pliability of the balloon 9, it will adapt well to the shape of the blood vessel 18, so that the latter is subjected to a uniform load when being dilated.

Subsequently one allows the pressure inside the balloon 9 to drop, as a result of which it will regain its original shape. The catheter is advanced further into the body, until the balloon 10 will be positioned in the dilated section of the vessel. By now supplying medium under pressure to the balloon 10 via connector 8 and the interspace in between the tube-like elements 5, 6, which interspace is connected at the end of the outer tube-like element 5 in the form of a ring-shaped opening 20 with the inside of the balloon 10, this balloon 10 and consequently the stent 13 will be expanded. This situation has been illustrated in figure 3.

As said before, the balloon 10 is less pliable. The expansion is therefore less determined by possible resistance, so that a uniform expansion of the stent is achieved.

Next one allows the pressure in the balloon 10 to drop again, as a consequence of which the balloon 10 regains its original shape with small diameter. The catheter can now be withdrawn whereby the stent 13 remains behind in the blood vessel 18 in order to support the wall thereof.

## Claims

1. Catheter comprising a tube-like basic body (4) with a proximal and a distal end, at least two balloon members , a relatively proximal (10) and a relatively distal balloon member (9), arranged close to the distal end which are connected with connecting members at the proximal end via lumens (16,17) in the basic body (4), wherein the relatively proximal balloon member (10) carries a compressed stent (13), **characterized in that**, the relatively distal balloon member (9) is more pliable than the relatively proximal balloon member (10) carrying the stent (13).

2. Catheter as claimed in claim 1, wherein the different degrees of pliability are a result of the fact that the wall thickness of the balloon members differs.

3. Catheter as claimed in claim 1, wherein the different degrees of pliability are a result of the fact that the balloon members have been manufactured of different materials.

4. Catheter as claimed in one of the previous claims, wherein the less pliable balloon member has a greater pressure resistance than the more pliable balloon member.

## Patentansprüche

1. Katheter, aufweisend einen rohrartigen Grundkörper (4) mit einem proximalen und einem distalen Ende, wenigstens zwei Ballonteile, ein relativ proximales (10) und ein relativ distales Ballonteil (9), die nahe dem distalen Ende angeordnet sind, welche über Lumen (16, 17) in dem Grundkörper (4) mit Anschlussteilen an dem proximalen Ende verbunden sind, wobei das relativ proximale Ballonteil (10) einen komprimierten Stent (13) trägt, **dadurch gekennzeichnet, dass** das relativ distale Ballonteil (9) biegsamer als das relativ proximale Ballonteil (10) ist, das den Stent (13) trägt.

2. Katheter nach Anspruch 1, wobei die unterschiedlichen Grade an Biegsamkeit ein Ergebnis der Tatsache sind, dass die Wanddicke der Ballonteile differiert.

3. Katheter nach Anspruch 1, wobei die unterschiedlichen Grade an Biegsamkeit ein Ergebnis der Tatsache sind, dass die Ballonteile aus unterschiedlichen Materialien hergestellt wurden.

4. Katheter nach einem der vorhergehenden Ansprüche, wobei das weniger biegsame Ballonteil einen größeren Druckwiderstand als das mehr biegsame Ballonteil hat.

## Revendications

1. Cathéter comprenant un corps de base (4) tubulaire avec une extrémité proximale et une extrémité distale, au moins deux éléments de ballonnet, un élément de ballonnet relativement proximal (10) et un élément de ballonnet relativement distal (9), disposés à proximité de l'extrémité distale, qui sont reliés par des éléments de connexion à l'extrémité proximale par le biais de lumières (16, 17) dans le corps de base (4), dans lequel l'élément de ballonnet (10) relativement proximal porte un stent comprimé (13), **caractérisé en ce que** l'élément de ballonnet (9) relativement distal est plus flexible que l'élément de ballonnet relativement proximal (10) portant le stent (13).

2. Cathéter selon la revendication 1, dans lequel les différents degrés de flexibilité sont un résultat du fait que l'épaisseur de paroi des éléments de ballonnets diffère.

3. Cathéter selon la revendication 1, dans lequel les différents degrés de flexibilité résultent du fait que les éléments de ballonnets ont été fabriqués avec des matériaux différents.

4. Cathéter selon l'une quelconque des revendications précédentes, dans lequel l'élément de ballonnet moins flexible a une résistance à la pression plus grande que l'élément de ballonnet plus flexible.
